Europäisches Patentamt

European Patent Office

Office européen des brevets

(11) Publication number: **0 079 145**
**B1**

(12) **EUROPEAN PATENT SPECIFICATION**

(45) Date of publication of patent specification: **09.10.85**

(21) Application number: **82305564.5**

(22) Date of filing: **19.10.82**

(51) Int. Cl.⁴: **G 01 N 33/571**

(54) **Reagent for use in diagnosis of syphilis and preparation thereof.**

(30) Priority: **23.10.81 JP 168646/81**

(43) Date of publication of application:
**18.05.83 Bulletin 83/20**

(45) Publication of the grant of the patent:
**09.10.85 Bulletin 85/41**

(84) Designated Contracting States:
**DE FR GB IT NL**

(56) References cited:
**EP-A-0 038 150**
**DE-A-2 429 231**
**GB-A-1 577 131**
**US-A-4 288 426**

(73) Proprietor: **FUJIREBIO KABUSHIKI KAISHA also
trading as FUJIREBIO INC.
6-7, Shimoochiai 4-chome Shinjuku-ku
Tokyo 161 (JP)**

(72) Inventor: **Sato, Takashi
A-310, 20, Nakaarai 3-chome
Tokorozawa-shi Saitama-ken (JP)**
Inventor: **Kubo, Emiko
766, Zooshiki 3-chome
Higashiyamato-shi Tokyo (JP)**

(74) Representative: **Silverman, Warren et al
HASELTINE LAKE & CO. Hazlitt House 28
Southampton Buildings Chancery Lane
London WC2A 1AT (GB)**

Courier Press, Leamington Spa, England.

## Description

This invention relates to an improved reagent for use in the TPHA test (*Treponema pallidum* hemagglutination test) which is a sero-diagnostic test for syphilis, and to a process for the production thereof. The TPHA test is carried out by hemagglutination of the antigen which is obtained from a culture of pathogenic *Treponema pallidum* Nichols (hereinafter referred to as TP) and which is sensitized on carrier particles such as mammalian red blood cells when in the presence of the corresponding antibody.

Prior to development of the TPHA test, the STS (Sorological Test for Syphilis) method was employed for the diagnosis of syphilis. However, this method is insufficiently specific. Accordingly, various methods utilising antigen-antibody reaction between the antigen of TP cells and the antibody in the blood serum of a patient were developed. For instance, the so-called FTA test (fluorescent treponema antibody test) was developed at an early stage. In this FTA test, the antibody in the serum of a patient is first reacted with the antigen of TP cells, and the immuno complex which is the product of the above antigen-antibody reaction is detected by using anti gamma globulin which is labelled with a fluorescent material. This FTA test has, however, not found widespread use because of its complicated procedure.

On the other hand, the aforementioned TPHA test which was developed by T. Tomizawa et al., is widely employed because of its sensitivity, specificity, and simplicity of procedure. Hence, at the present time, this TPHA test has become a standard diagnostic test for syphilis. However, a weakness of this test is that detection of primary syphilis, i.e. syphilis at a primary stage, generally within 2 or 3 months of intection is uncertain and the TPHA test often gives a negative result when primary syphilis is present. Accordingly, in order to ensure the detection of primary syphilis, it is necessary that the STS method be employed together with the TPHA test.

It is an object of the invention to provide a diagnostic reagent which can be used in the TPHA test to yield reliable results even in the presence of primary syphilis.

According to the present invention, there is provided a reagent for use in the diagnosis of syphilis by a procedure which utilises the homagglutination, in the presence of an antibody, of an antigen which is obtained from a culture of pathogenic *Treponema pallidum,* preferably the Nichols strain, which reagent comprises carrier particles sensitised by having bonded thereto a said antigen which is devoid of fractions of said culture having a specific gravity lower than 1.01.

This invention is based on the discovery that when antigen sensitised on a carrier is used for the measurement of immunoglobulin using indirect passive homagglutination in the TPHA test, if a particular impurity is removed from a mixture containing antigen prior to sensitisation of carrier particles, the antigen sensitised on the carrier particles can be reliably used to detect primary syphilis. More particularly, it has been observed that when conventional antigen is sensitised on a carrier, proteinic impurities are also immobilised on the carrier in quantity. In the TPHA test, more than 90% of the proteinic impurities are derived from the testes of rabbit in which TP is cultured. These proteinic impurities have been found to occur generally in fractions having a specific gravity of less than 1.01 and accordingly these are removed prior to the sensitisation of the antigen.

The present invention is based more particularly on the discovery that the low sensitivity of the conventional TPHA test against the primary antibody (Ig—M) compared to the sensitivity against the later antibody (Ig—G) is caused by particular impurities contained in the extracts of the TP. But for the practical problems otherwise involved, it would not be necessary for the above-mentioned particular impurities which are contained in the fractions having a specific gravity of lower than 1.01 to be removed completely, and it would be sufficient for them to be removed only to the extent that the remaining impurities do not substantially interfere with the antigen-antibody reaction between the antigen-sensitised carrier and the Ig—M antibody of syphilis. The removal of impurities may be carried out either before or after the destruction of microbial cells. However, the removal of impurities is preferably carried out before the destruction of the microbial cells by the density gradient method.

When carrying out the present invention, it is necessary to remove those proteinic impurities whose specific gravities are lower than 1.01. It is preferable that the fractions having specific gravities lower than 1.05 are removed. The fractions having specific gravities higher than 1.20 are also preferably removed.

The protein content of the solution of the antigen which is to be used for sensitisation and which is obtained from the testes of rabbits in conventional manner is more than 0.6 mg per $10^9$ TP cells. In contrast, the protein content of the antigen solution for sensitisation employed in the present invention is less than 0.3 mg per $10^9$ TP cells, and usually less than 0.1 mg per $10^9$ TP cells. The above protein contents were measured by the Lowry's method (Lowry et al., J. Biol. Chem., Vol. 193, pp 265—275 (1951)), and the number of TP cells was determined by direct counting of TP cells in the sample placed on a bacteria counter (manufactured by C. A. Hauser & Son) using a dark-field microscope.

The solution of antigen which is to undergo sensitisation may be obtained by a conventional method. The TP strain which is to be used for inoculation will usually be the WHO pathogenic standard strain Nichols, although this invention is also applicable to other TP strains employed in the TPHA test. The WHO pathogenic standard *Treponema pallidum Nichols* strain is available, for example from CDC (Center for Disease Control, Public Health Service, U.S. Department of Health, Education and Welfare, Atlanta, Georgia). To produce samples of the strains, rabbits are inoculated with the TP strain in the testes and housed for 11 to 14 days. After the cells of the strain have undergone cultivation, multiplied cells are

2

collected according to the method of Miller et al. (Miller et al., Journal of Immunology, Vol. 96, p 450 (1966)). An abstract of Miller's method is as follows:

After the cultivation, the testes are minced, and multiplied cells are extracted from the minced testes by using a mixed solution formed from equal volumes of inactivated normal rabbit serum and 1/7 M saline solution. The extracts are centrifuged at 200×g, and the solid material consisting of large particles constituted by testis tissue is removed. The supernatant is centrifuged again at 19,000×g for 90 minutes, and the separated-out solid matter containing the multiplied cells is recovered. Then, this solid matter is washed three times with chilled 0.075 M sodium oxalate.

In the conventional method for carrying out the TPHA test, the washed solid matter is suspended in a suitable buffer solution, and the TP microbial cells are destroyed by homogenisation, by ultrasonic processing, by treatment with surfactant, by treatment with enzyme, by autolysis, or by freezing and thawing. When the removal of the particular impurities previously described is carried out prior to the destruction of TP cells, the fractions of TP cells from which the particular impurities have been removed are preferably washed with a buffer solution by means of centrifugation etc., and then the TP cells are destroyed.

The removal of the fractions having the specific gravity of less than 1.01 may be carried out by any convenient fractionation technique, such as density gradient, gel filtration, ultracentrifugation, ultrafiltration, fractionation using ammonium sulphate, and electrophoresis. Of these methods, density gradient and ultracentrifugation are most suitable for use in the present invention. The removal of the above fractions may also be carried out by adsorption of the antigen on the corresponding antibody which is immobilised on a carrier.

The suspension of the destroyed cells from which the undesired fractions have been removed may be diluted with a buffer solution and then, antigen in the suspension is used to sensitise carrier particles, such as sheep red blood cells, in conventional manner.

The carrier particles to be used are not limited to sheep red blood cells, and include any particles usable as a carrier for indirect passive hemagglutination using an antigen-antibody reaction, including various mammalian erythrocytes, microbial cells, polystyrene latex, and gelatin particles.

The antigen-sensitised carrier is optionally lyophilised and is then combined with any of the reagents employed in the diagnosis of syphilis by the TPHA method, such as a standard serum, an unsensitised carrier, an absorbing solution, a diluent, a reconstituting solution, etc., to yield a complete reagent for use in diagnosis of syphilis.

It is found with antigen sensitised carriers embodying this invention that non-specific reactions are slightly increased. However, if one of the aforementioned reagents employed in the TPHA method, whether or not that to which the antigen-sensitised carrier is added, contains the antigen derived from a strain belonging to the genus *Treponema* which lives in the human body or an animal body is employed as one of the above reagents, the above non-specific reactions can completely or almost be eliminated. The solution to which the antigen is added is for example the absorbing solution, the diluent or the reconstituting solution. Such strains include *Treponema denticola, Treponema mucosum, Treponema orale, Treponema scoliodontum, Treponema vincentii, Treponema macrodentium, Treponema trimerontium, Treponema buccalis, Treponema enterogyratum, Treponema paraluis-cuniculi, Treponema hyos,* and *Treponema penortha*. The solution containing the above antigen may be produced by a conventional method for producing reagent for the TPHA tust. For example, a culture broth of one of the above strains is centrifuged at 10,000×g, and the supernatant is separated off. The supernatant is heated to 100°C, and deposits are removed to obtain a culture filtrate. The material separated off in the centriguation step is added to the culture filtrate to obtain the solution containing the above antigen. In this case, as with the cells used to produce the antigen to be sensitised on carrier particles, the cells of the strain may be destroyed by homogenisation or by ultasonic processing.

The antigen-sensitised carrier embodying this invention indicates both specifically and reliably positively the existance both of well-established syphilis in a patient and the existence of primary syphilis, i.e. within one month of infection. This antigen-sensitised carrier makes the diagnosis of syphilis easy, and in particular avoids the need to carry out the more complicated STS method.

The present invention is further illustrated by the following examples.

Example 1

A sample of the WHO pathogenic standard *Treponema pallidum Nichols* strain from the National Institute of Health, Ministry of Health & Welfare (Japan) was cultured, and the multiplied cells were suspended in a concentration of $6.0 \times 10^7$ cells/ml.

Each test is of 10 rabbits was inoculated with one ml of the suspension, and cultured for 12 days. All the testes were removed from the rabbits, and minced. Multiplied cells were extracted from the minced testes by using 1000 ml of a mixed solution consisting of equal volumes of inactivated normal rabbit serum and 1/7 M saline solution.

The extracts were centrifuged at 200×g from 10 minutes, and precipitated material was removed. The supernatant was centrifuged again at 19,000×g for 90 minutes, and TP cells were separated out.

The precipitates thus formed containing TP cells were washed three times with chilled 0.075 M sodium oxalate, and were suspended in 10 ml of 1/15 M phosphate buffer solution of pH 6.4. The number of TP cells

in the suspension was counted, and then the suspension was diluted so that the concentration of the TP cells was $2\times10^9$/ml, thereby providing a crude TP antigen solution.

Sodium diatrizoate solutions of 60%, 37.5%, 25.0% and 19.0% (w/v %) concentration thereby providing a density gradient were taken up in a 15 ml cellulose tube, and one ml of the crude TP antigen solution was provided on the topmost layer of the sodium diatrizoate solution. The cellulose tube was centrifuged at 25,000 rpm ($76,000\times$g) at 20°C for 2 hours, and 15 fractions each of one ml were successively withdrawn from the top of the layers.

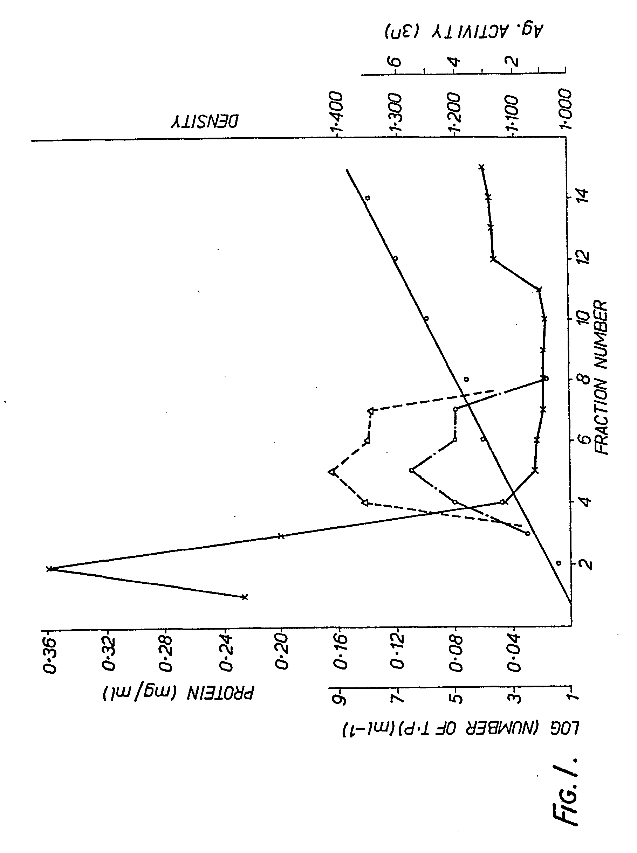

The number of TP cells in and the protein concentration of the crude TP antigen solutions obtained were measured and the numbers of TP cells in, the protein concentrations of the densities of and the antigenic activities of each fraction were measured, the results obtained are shown in Figure 1 of the accompanying drawings, cell numbers being shown by triangles, protein concentrations by crosses, densities by Circles (open) and antigenic activities by solid circles.

The various values were determined as follows:

The numbers of TP cells were determined by direct counting of TP cells in each sample placed on a bacteria counter (manufactured by C. A. Hauser & Son) using a dark-field microscope.

The densities were determined by weighing each 100 µl of sample.

The protein concentrations were determined by Lowry's method in which sodium carbonate solution containing sodium potassium tartrate and copper sulphate was added to each sample, and the mixture was allowed to stand for 10 minutes at room temperature. Then, Folin's reagent was added to the mixture, and after more than 30 minutes, the mixture was colourimetrically determined at 750 nm.

To determine the antigenic activity of TP, each sample was ultrasonically processed in order to destroy TP cells. Then, 20 µl of the thus processed sample were placed in the left end well of a micro titre plate, and diluted twice by twice with the solution of antibody against TP antigen on the micro titre plate. The micro titre plate was kept at 37°C for 30 minutes, and antigen-sensitised carrier was added to each well. The antigenic activity of TP is the dilution ratio when hemagglutination appears.

Fractions Nos. 4 to 7 in Figure 1 were combined to obtain one ml of antigen solution for sensitisation testing. The number of TP cells in this antigen solution was $1.91\times10^9$, and the protein content was 0.116 mg. In contrast, the number of TP cells in the crude TP antigen solution was $2.0\times10^9$ and the protein content was 1.213 mg.

When using this antigen solution for sensitisation testing, the antigen was taken up on sheep red blood cells, and the sensitised cells were lyophilised.

Both this lyophilised matter and an antigen sensitised carrier which was prepared from the crude TP antigen solution as used conventionally were used in the diagnosis of syphilis. There were employed three serums ($G_1$, $G_2$, $G_3$) obtained from patients having syphilis who had been undergoing sufficient treatment and three serums ($M_1$, $M_2$, $M_3$) obtained from the patients having primary syphilis infected after 3 to 4 weeks. The former three serums contained only Ig—G antibody, and the latter three serums contained only Ig—M antibody.

The results obtained are shown in Table 1.

TABLE 1

| | Primary syphilis | | | Late syphilis | | |
|---|---|---|---|---|---|---|
| | $M_1$ | $M_2$ | $M_3$ | $G_1$ | $G_2$ | $G_3$ |
| The Product of the Invention | + ($\times$320) | + ($\times$320) | + ($\times$160) | + ($\times$640) | + ($\times$320) | + ($\times$160) |
| The Conventional Product | — ($\times$40) | — ($\times$40) | — ($\times$20) | + ($\times$2560) | + ($\times$640) | + ($\times$320) |

+...positive, −...negative

Figures in parentheses indicate antibody titre. Positive means an antibody titre of more than 80.

Example 2

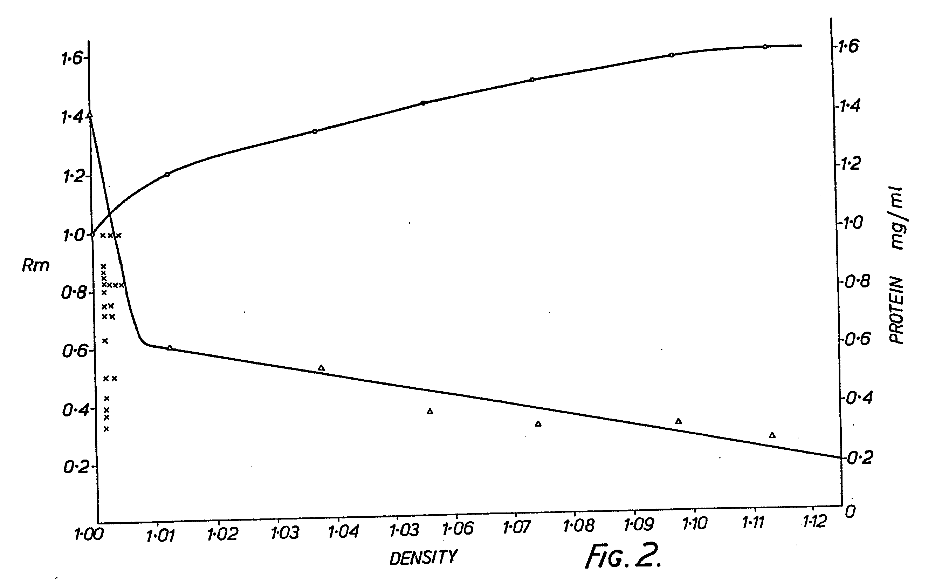

25% w/v sodium diatrizoate solutions (d=1.161) were placed in centrifuge tubes, and sodium diatrizoate solutions having the various specific gravities shown in Figure 2 of the accompanying drawings were applied thereto.

The crude TP antigen solution obtained in Example 1 was applied to each upper layer, and each centrifuge tube was centrifuged. The middle fraction formed between the two sodium diatrizoate solutions was withdrawn.

The protein concentration of each middle fraction was measured by Lowry's method. Each middle fraction was sensitised on sheep red blood cells by the conventional tannic acid method, and the titres of the antigen-sensitised sheep cells were measured according to the TPHA test by using serums containing only Ig—M antibody and only Ig—G antibody respectively. Both serums had been previously adjusted by mixing the serums of several patients with syphilis to provide equal titres when the TPHA test was carried out using the antigen-sensitised sheep cells prepared by using the crude TP antigen solution to sensitise sheep red blood cells.

The results obtained are shown in Figure 2 of the accompanying drawings. In the Figure, the abscissa indicates the specific gravity of the sodium diatrizoate solution of the upper layer, and the left-hand ordinate indicates the ratio ($R_M$) of the titre of the antigen-sensitised sheep cells against Ig—M antibody to the titre against Ig—G antibody, the ratio values being shown as circles. The right-hand ordinate indicates the concentrations plotted as triangles.

As can be seen from Figure 2, the sensitivity of the antigen-sensitised sheep cells with respect to the Ig—M syphilis antibodies increases considerably greater than that against Ig—G syphilis antibodies on removal of the fractions having lower specific gravities, particularly the fractions having a specific gravity of lower than 1.01.

The TPHA test was also carried out on the above serums using several conventional materials. The results obtained are shown adjacent the left-hand ordinate of Figure 2 by the crosses.

In order to illustrate the effect of a solution containing an antigen derived from a strain belonging to genus *Treponema* which lives in the human body or an animal body on the TPHA test, the following experiment was carried out.

Experiment

20 ml of a culture broth containing *Treponema mucosum* E-21 supplied by Nippon Dental University and in concentration of $1.4 \times 10^8$ cells/ml were centrifuged at $10,000 \times g$ at 4°C for 30 minutes, and the solid material containing microbial cells was separated from the supernatant. The supernatant was heated for 30 minutes in a water bath at 100°C. After cooling, the supernatant was filtered using Whatman No. 2 filter paper, and a culture filtrate was obtained.

The solid material from the centrifugation was suspended in A-solution containing.

| | |
|---|---|
| $Na_2HPO_4 \cdot 12H_2O$ | 20.7 g/l |
| $KH_2PO_4$ | 2.3 ,, |
| NaCl | 4.3 ,, |
| Gum arabic | 2.5 ,, |
| $NaN_3$ | 1.0 ,, |
| Healthy rabbit serum | 10 ml/l |
| 10% Tween 80 (registered Trademark) | 1 ,, |

The suspension was ultrasonically processed at 20 kHz for 10 minutes so that the microbial cells were destroyed. The thus processed suspension was mixed with the culture filtrate, and the total volume of the mixture was adjusted to 40 ml by addition of A-solution. The mixture obtained was named E-solution.

20 ml of a culture broth containing *Treponema denticola* S-173 also supplied by Nippon Dental University and in a concentration of $1.4 \times 10^8$ cells/ml were treated in the same manner and 40 ml of the final mixture obtained was named S-solution.

Both E-solution and the S-solution were diluted 10 times with the A-solution, and the diluted solutions were mixed with each other. The diluted mixture so produced was named ES-solution.

0.06 gram of wet cells of *Treponema phagedenis Reiter* was added to 1 l of the A-solution, and the mixture obtained was named B-solution.

Both the E-solution and the S-solution were diluted 10 times with the B-solution, and the diluted solutions were mixed with each other. The mixture obtained was named BES-solution.

The TPH test was then carried out using the lyophilised antigen-sensitised sheep cells obtained in Example 1, and one of the above ES-, BES-, and B-solutions. In this test, the chosen solution was employed as the diluent, the reconstituting solution, and the absorbing solution, 560 serum samples from healthy persons were diluted 10 times with the above solution, and the diluted serums were employed as the samples.

The results of the TPHA test are shown in Table 2.

TABLE 2

|  | The number of persons detected as positive (%) |
|---|---|
| ES-solution | 10 (1.8%) |
| BES-solution | 2 (0.36%) |
| B-solution (control) | 50 (8.9%) |

**Claims**

1. A reagent for use in the diagnosis of syphilis by a procedure which utilises the hemagglutination, in the presence of an antibody, of an antigen which is obtained from a culture of pathogenic *Treponema pallidum*, preferably the Nichols strain, which reagent comprises carrier particles sensitised by having bonded thereto a said antigen characterised in that said antigen is devoid of fractions of said culture having a specific gravity lower than 1.01.

2. A reagent as claimed in claim 1, wherein the antigen is free of fractions of said culture having specific gravities outside the range of 1.05 to 1.20.

3. A reagent as claimed in claim 1 or 2, wherein said carrier particles are mammalian erythrocytes, microbial cells, polystyrene latex or gelatin particles.

4. A reagent as claimed in claim 3, wherein said carrier particles are sheep red blood cells.

5. A reagent as claimed in any one of the preceding claims, when forming part of a kit of materials for use in the diagnosis of syphilis, which kit additionally comprises a standard serum, an unsensitised carrier, an absorbing solution, a diluent and a reconstituting solution.

6. A reagent as claimed in any one of claims 1 to 4, when forming part of a kit of materials for use in the diagnosis of syphilis, which kit additionally comprises a liquid medium selected from an absorbing solution, a diluent and a reconstituting solution which contains an antigen derived from a strain belonging to genus *Treponema* which lives in the human body or an animal body.

7. A reagent as claimed in claim 6, wherein said liquid medium contains an antigen derived from a strain belonging to genus *Treponema* which is *Treponema denticola Treponema mucosum, Treponema orale, Treponema scoliodontum, Treponema vincentii, Treponema macrodentium, Treponema trimerontium, Treponema buccalis, Treponema enterogyratum, Treponema paraluis-cuniculi, Treponema hyos* and *Treponema penortha*.

8. A process for the production of sensitised carrier particles for use in the diagnosis of syphilis comprising depositing antigen obtained from a culture of a pathogenic *Treponema pallidum*, preferably the Nichols strain, on carrier particles, in which process solution fractions of a said culture having specific gravities lower than 1.01 are removed from said antigen-containing solution prior to using the antigen-containing solution in sensitisation of the carrier particles by deposition thereon.

9. A process as claimed in claim 8, wherein said fractions are removed by the density gradient method.

10. A process as claimed in claim 8 or 9, wherein the antigen solution used for sensitisation contains less than 0.3 mg, preferably less than 0.1 mg, of protein per $10^9$ *Treponema pallidum* cells.

**Patentansprüche**

1. Reagens zur Verwendung für die Diagnose von Syphilis bei einem auf der Hämagglutination beruhenden Verfahren in Gegenwart eines Antikörpers sowie eines Antigens, welches gewannen wurde aus der Kultur von pathogenen Treponema pallidum, vorzugsweise der Nichols- Art, und auf in dem Reagens vorhandene Trägerteilchen zu deren Sensibilisierung gebunden ist, dadurch gekennzeichnet, daß das besagte Antigen frei von Fraktionen der Kultur ist, die ein spezifisches Gewicht unter 1,01 haben.

2. Reagens gemäß Anspruch 1, wobei das Antigen frei ist von Fraktionen der Kultur, die ein spezifisches Gewicht außerhalb des Bereichs von 1,05 bis 1,20 haben.

3. Reagens gemäß Anspruch 1 oder 2, worin die Trägerteilchen sind: Erythrozyten von Säugetieren, mikrobische Zellen, Polystyrol- Latex oder Gelatine- Teilchen.

4. Reagens gemäß Anspruch 3, wobei die Trägerteilchen von Schafen stammende rote Blutzellen sind.

5. Reagens gemäß einem jeden der vorangehenden Ansprüche, welches Bestandteil eines Materialsatzes zur Verwendung für die Diagnose von Syphilis ist, wobei der Ausstattungssatz zusätzlich enthält ein Standard- Serum, einen unsensibilisierten Träger, eine Absorbtionslösung, ein Verdünnungsmittel und Wiederaufbaulösung.

6. Reagens gemäß einem jeden der Ansprüche 1 bis 4, welches Bestandteil eines Materialsatzes zur Verwendung für die Diagnose von Syphilis ist, wobei der Ausstattungssatz ein aus einer absorbierenden Lösung, einem Verdünnungsmittel oder einer Wiederausbaulösung ausgewähltes flüssiges Medium

6

enthält, welches einen Gehalt an einem Antigen aufweist, das von einer im menschlischen oder tierischen Körper lebenden Art aus der Gattung Treponema stammt.

7. Reagens gemäß Anspruch 6, worin das besagte flüssige Medium ein Antigen enthält von einer Art der Gattung Treponema, und zwar Treponema denticola, Treponema mucosum, Treponema orale, Treponema scoliodontum, Treponema vincentii, Treponema macrodentium, Treponema trimerontium, Treponema buccalis, Treponema enterogyratum, Treponema paraluis-cuniculi, Treponema hyos und Treponema penortha.

8. Verfahren zur Herstellung von sensibilisierten Trägerteilchen zur Verwendung bei der Diagnose von Syphilis durch Aufbringung eines Antigens, das aus einer Kultur von pathogenen Treponema pallidum, vorzugsweise der Nichols- Art gewonnen wurde, auf Trägerteilchen, wobei aus der Kulturlösung Fraktionen mit einem spezifischen Gewicht unter 1,01 entfernt werden und danach die Antigen enthaltende Lösung auf die Träger zu deren Sensibilisierung aufgebracht wird.

9. Verfahren gemäß Anspruch 8, wobei die besagten Fraktionen durch die Dichtegradient- Methode entfernt wurden.

10. Verfahren gemäß Anspruch 8 oder 9, wobei die zur Sensibilisierung verwendete Antigenlösung weniger als 0,3 mg, besonders weniger als 0,1 mg Protein pro $10^9$ Treponema-Zellen enthält.

## Revendications

1. Réactif à employer dans le diagnostic de la syphilis par un procédé qui utilise l'hémagglutination, en présence d'un anticorps, d'un antigène qui est obtenu à partir d'une culture de *Treponema pallidum* pathogène, préférablement la souche de Nichols, lequel réactif comprend des particules porteuses sensibilisées par leur liaison au dit antigène, caractérisé par le fait que ledit antigène est dépourvu de fractions de ladite culture qui ont une densité inférieure à 1,01.

2. Réactif selon la revendication 1, dans lequel l'antigène est exempt de fractions de ladite culture qui ont des densités non comprises entre 1,05 et 1,20.

3. Réactif selon la revendication 1 ou 2, dans lequel lesdites particules porteuses sont des érythrocytes de mammifère, des cellules microbiennes, des particules de gélatine ou de latex de polystyrène.

4. Réactif selon la revendication 3, dans lequel lesdites particules porteuses sont des globules rouges de mouton.

5. Réactif selon l'une quelconque des revendications précédentes, lorsqu'il fait partie d'un nécessaire de produits à employer dans le diagnostic de la syphilis, lequel nécessaire comprend en outre un sérum-étalon, un porteur non sensibilisé, une solution absorbante, un diluant et une solution de reconstitution.

6. Réactif selon l'une quelconque des revendications 1 à 4, lorsqu'il fait partie d'un nécessaire de produits à employer dans le diagnostic de la syphilis, lequel nécessaire comprend en outre un milieu liquide choisi à partir d'une solution absorbante, un diluant et une solution de reconstitution qui contient un antigène dérivé d'une souche appartenant au genre *Treponema* qui se développe dans l'organisme humain ou dans un organisme animal.

7. Réactif selon la revendication 6, dans lequel ledit milieu liquide contient un antigène dérivé d'une souche appartenant au genre *Treponema* qui est *Treponema denticola, Treponema mucosum, Treponema orale, Treponema scolidontum, Treponema vincentii, Treponema macrodentium, Treponema trimerontium, Treponema buccalis, Treponema enterogyratum, Treponema paraluis-cuniculi, Treponema hyos* et *Treponema penortha*.

8. Procédé pour la production de particules porteuses sensibilisées à employer dans le diagnostic de la syphilis, comprenant le dépôt sur des particules porteuses d'un antigène obtenu à partir d'une culture d'un *Treponema pallidum* pathogène, préférablement la souche de Nichols, dans lequel procédé des fractions de solution d'une dite culture qui ont des densités inférieures à 1,01 sont éliminées de ladite solution contenant un antigène, avant d'employer la solution contenant un antigène pour la sensibilisation des particules porteuses en la déposant sur celles-ci.

9. Procédé selon la revendication 8, dans lequel on sépare lesdites fractions par la méthode de gradient de densités.

10. Procédé selon la revendication 8 ou 9, dans lequel la solution d'antigène utilisée pour la sensibilisation contient moins de 0,3 mg, préférablement moins de 0,1 mg de protéine par $10^9$ cellules de *Treponema pallidum*.

Fig. I.

FIG. 2.

0 079 145